# EUROPÄISCHE PATENTANMELDUNG

(11) **EP 1 529 552 A1**
(43) Veröffentlichungstag der Anmeldung: **11.05.2005**
(21) Anmeldenummer: 04026128.1
(22) Anmeldetag: 04.11.2004
(51) Int. Cl.: A61N 5/06

(54) **Vorrichtung zum Ermitteln der optimalen Bräunungsdauer von Personen in Solarien und Solarium**

(30) Priorität: 05.11.2003 DE 10352203
(71) Anmelder: UV-Power Licht GmbH, 49716 Meppen (DE)
(72) Erfinder: Schaffron, Günter, 49716 Meppen (DE)
(74) Vertreter: Patentanwälte Hauck, Graalfs, Wehnert, Döring, Siemons, Schildberg

(57) **Zusammenfassung**

Es werden eine Vorrichtung zum Ermitteln der optimalen Bräunungsdauer von Personen in Solarien und ein zugehöriges Solarium beschrieben. Die Vorrichtung umfaßt einen UV-Sensor zum Bestimmen der Strahlungsintensität des Solariums und eine Steuerungseinheit, die eine Dateneingabeeinheit, eine Datenausgabeeinheit und eine Zentraleinheit umfaßt. Mit Hilfe der Datenausgabeeinheit wird die optimale Bräunungsdauer in Abhängigkeit von der UV-Strahlungsintensität und personenspezifischen Daten angezeigt.

## Beschreibung

Die vorliegende Erfindung betrifft eine Vorrichtung zum Ermitteln der optimalen Bräunungsdauer von Personen in Solarien.

Solarien sind allgemein bekannt und dienen zum künstlichen Bräunen der Haut durch die Einwirkung von UV-Strahlung. Hierzu besitzen die Solarien eine entsprechende Zahl von diese Strahlung abgebenden UV-Röhren. Der hier verwendete Begriff "Solarien" soll Solarien im weitesten Sinne abdecken, d.h. Sonnenbänke, Sonnenduschen etc., d.h. solche in liegender und stehender Bauweise.

Derartige Solarien sind üblicherweise in Sonnenstudios aufgestellt. Um eine optimale Bestrahlung zu erzielen, ist eine Reihe von bestrahlungsspezifischen aber auch personenspezifischen Parametern zu beachten. So spielen der Hauttyp und der Vorbräunungsgrad der Haut eine Rolle. Die entsprechenden UV-Leuchtstoffröhren der Solarien geben UV-A-Strahlung und UV-B-Strahlung ab. Beide Strahlungen lösen, vom Körper aufgenommen, eine Reihe von fotobiologischen und/oder fotochemischen Prozessen aus (Melaninaktivierung, Bräunung, Sonnenbrand, Hautalterung etc.). Die Intensität der UV-A-Strahlung und der UV-B-Strahlung wird als Bestrahlungsstärke gemessen. Die Bestrahlungsstärke ist das Verhältnis von Strahlenfluß zu einer bestrahlten Fläche. Die Meßeinheit ist W/m². Bei der biologischen Bestrahlungsstärke handelt es sich um die Strahlung, die auf der menschlichen Haut eine entsprechende Wirkung erzielt. Hierbei unterscheidet man die Direkt- oder Sofortpigmentierung, die verzögerte Tiefenpigmentierung und die erythemwirksame Bestrahlungsstärke. Die erythemwirksame Bestrahlungsstärke ist wohl der wichtigste Wert, da sie die Besonnungszeit bestimmt. Sie erzeugt auch den Sonnenbrand auf der Haut. Die erythemwirksame Bestrahlung liegt in einem Wellenbereich von 280nm - 400 nm. Der Hauptanteil dieser Strahlung liegt im UV-B-Bereich von 280 nm - 315 nm.

Zur Ermittlung der optimalen Bräunungsdauer von Personen hat man bisher aufgrund der vorstehend angegebenen strahlungsspezifischen und personenspezifischen Daten individuelle Besonnungspläne aufgestellt. In der Praxis hat dabei der Kunde eines Sonnenstudios vom Bedienungspersonal entsprechende Zeitangaben erhalten. Die zeitaufwendige Ermittlung dieser Angaben ist dabei in vielen Fällen ungenau und fehlerhaft gewesen, so daß sich in vielen Fällen keine optimalen Bräunungsdauern ergeben haben.

Neben der vorstehend beschriebenen Vorgehensweise ist es auch bereits bekannt, kundenspezifische und gerätespezifische Daten zu sammeln und auf einem Datenträger zu speichern. Der Kunde kann dann mit Hilfe dieses Datenträgers, der die vom Hersteller angegebenen bestrahlungsspezifischen Parameter eines entsprechenden Gerätes enthält, die gewünschte optimale Bräunungszeit abrufen. Diese Vorgehensweise bringt insofern Probleme mit sich, als daß hiermit keine aktuellen Strahlungsintensitätsdaten erfaßt werden können, so daß die Ergebnisse verfälscht sein können.

Der vorliegenden Erfindung liegt die Aufgabe zugrunde, eine einfach ausgebildete und einfach und rasch handhabbare Vorrichtung zu schaffen, mit der der Benutzer von Solarien seine individuelle aktualisierte und optimierte Bräunungszeit ermitteln kann.

Diese Aufgabe wird erfindungsgemäß durch eine Vorrichtung mit den folgenden Bestandteilen gelöst:
a. einem UV-Sensor zum Bestimmen der Strahlungsintensität des Solariums; und
b. einer Steuerungseinheit enthaltend
c. eine Dateneingabeeinheit zum Eingeben von Eigenschaften (Vorbräunung, Hauttyp etc.) einer das Solarium benutzenden Person kennzeichnenden Daten;
d. eine Datenausgabeeinheit zum Ausgeben von die optimale Bräunungsdauer kennzeichnenden Daten; und
e. eine Zentraleinheit zum Ansteuern des UV-Sensors und der Datenausgabeeinheit, zum Empfangen der Daten vom UV-Sensor und der Dateneingabeeinheit und zum Auswerten dieser Daten und Berechnen der Bräunungsdauerdaten.

Die erfindungsgemäß ausgebildete Vorrichtung basiert auf dem Messen eines gerätespezifischen Parameters, nämlich der UV-Strahlungsintensität des Solariums, und dem Erfassen von mindestens einem personenspezifischen Parameter, insbesondere von Vorbräunungsdaten, der das Solarium benutzenden Person. Über die Dateneingabeeinheit gibt die Person die Vorbräunungsdaten ein, die der Zentraleinheit zugeführt werden. Ferner wird von der Vorrichtung die Strahlungsintensität mit Hilfe des UV-Sensors gemessen, und die entsprechenden Daten werden ebenfalls der Zentraleinheit zugeführt. Diese Daten werden von der Zentraleinheit mit Hilfe einer geeigneten Software ausgewertet, und hieraus wird die optimale Bräunungsdauer der Person berechnet und über die Datenausgabeeinheit ausgegeben. Das Bestimmen der Strahlungsintensität mit Hilfe des UV-Sensors erfolgt vorzugsweise automatisch in bestimmten Zeitabständen, beispielsweise alle fünf Stunden, und die entsprechenden Meßdaten werden in einem Speicher gespeichert. Will eine Person nunmehr die optimale Bräunungsdauer ermitteln, liest die Zentraleinheit den aktuellen Strahlungsintensitätswert aus dem Speicher und benutzt diesen Wert zusammen mit den eingegebenen personenspezifischen Daten zur Ermittlung der optimalen Bräunungsdauer. Die Person erhält somit auf einfache und rasche Weise die gewünschten Daten. Diese Daten sind darüber hinaus relativ aktuell, da die UV-Strahlungsintensität im Betrieb des Solariums gemessen wird und keine gerätespezifischen Angaben des Herstellers verwendet werden, so daß auf diese Weise die entsprechenden Ergebnisse durch Alterungserscheinungen, Beschädigungen etc. der UV-Röhren der Solarien nicht verfälscht werden.

Der erfindungsgemäß verwendete UV-Sensor mißt die UV-Strahlungsintensität bzw. Bestrahlungsstärke in W/m². Vorzugsweise handelt es sich bei dem Sensor um einen Typ mit Erythemcharakteristik. Abhängig von der Strahlungsintensität gibt er analoge Spannungssignale ab, die vorzugsweise digitalisiert und der Zentraleinheit zugeführt werden.

Wie erwähnt, erfolgt die Auswertung der Meßdaten der UV-Sensors zusammen mit den eingegebenen personenspezifischen Daten über geeignete Software, die vorzugsweise die erythemwirksame Bestrahlungsstärke berücksichtigt, da diese einen wichtigen Wert für die Bestimmung der Besonnungszeit darstellt und auch den Sonnenbrand auf der Haut erzeugt. Für die Auswertung werden die entsprechenden maximal zulässigen Werte zugrunde gelegt, und hieraus werden unter Berücksichtigung der Vorbräunung und der gemessenen Strahlungsintensität optimierte Werte für die Bräunungsdauer ermittelt, und zwar beispielsweise für verschiedene Hauttypen. Natürlich ist es auch möglich, den Haupttyp oder andere mögliche personenspezifische Eigenschaften einzugeben und von der Zentraleinheit zur Bestimmung der optimierten Bräunungsdauer mit auswerten zu lassen.

Die erfindungsgemäße Vorrichtung besitzt vorzugsweise einen Speicher zum Speichern der gemessenen Starhlungsintensitätswerte. Auf diese Weise können permanent in zeitlichen Abständen Strahlungsintensitätsmessungen durchgeführt und die erzielten Ergebnisse abgespeichert werden. Will eine Person nunmehr die optimale Bräunungsdauer ermitteln, greift die Vorrichtung auf den jeweils aktuellen, im Speicher gespeicherten Meßwert zurück, so daß immer aktualisierte Gerätedaten zur Verfügung stehen. Die Zentraleinheit liest Werte in den Speicher ein und aus diesem aus.

Die Datenausgabeeinheit weist vorzugsweise eine optische Anzeige auf, und die Dateneingabeeinheit umfaßt vorzugsweise eine Tastatur. Besonders bevorzugt sind die Dateneingabeeinheit und Datenausgabeeinheit als Benutzerinterface mit Folientastatur und LCD-Display ausgebildet.

Die Zentraleinheit ist vorzugsweise als Microcontroller ausgebildet. Wie erwähnt, steuert die Zentraleinheit den Uv-Sensor vorzugsweise derart an, daß dieser in zeitlichen Abständen mehrfach die Strahlungsintensität mißt.

Die erfindungsgemäß ausgebildete Vorrichtung ist an ein entferntes Terminal anschließbar, das sich beispielsweise am Empfang eines Sonnenstudios befinden kann. Über dieses Terminal können vorzugsweise Daten eingegeben und abberufen werden.

Die Erfindung betrifft ferner ein Solarium, das eine Vorrichtung der vorstehend wiedergegebenen Art enthält. Bei diesem Solarium ist die Steuerungseinheit vorzugsweise in den Deckel des Solariums eingebaut oder an diesen angebaut. Sie ist somit von außen bedienbar, d.h. das Benutzerinterface (falls ein solches vorhanden ist) kann ohne Probleme vom Benutzer zur Ermittlung der optimalen Bräunungszeit genutzt werden. Was den UV-Sensor der erfindungsgemäßen Vorrichtung anbetrifft, so ist dieser vorzugsweise der höchstgelegenen UV-Röhre im Dekkel des Solarium zugeordnet, und zwar insbesondere über die Länge der Röhre etwa mittig, so daß die UV-Strahlungsintensität auf korrekte Weise erfaßbar ist.

Es versteht sich, daß die erfindungsgemäß ausgebildete Vorrichtung diverse weitere Bauteile (Stromquellenanschluß, AD-Wandler etc.) besitzt, die dem Fachmann geläufig sind und an dieser Stelle nicht im einzelnen aufgeführt werden müssen.

Es versteht sich, daß die von der erfindungsgemäßen Vorrichtung ermittelten optimalen bzw. empfohlenen Besonnungszeiten den empfohlenen MED-Einheiten entsprechen.

Die erfindungsgemäß ausgebildete Vorrichtung macht somit den Sonnenstudiokunden auf einfache Weise einen Vorschlag für die optimale Bräunungsdauer. Natürlich kann sich dieser Vorschlag auch auf die maximal erlaubte Bräunungsdauer beziehen. Der Vorschlag ist abhängig von der Strahlungsintensität des Solariums und von personenspezifischen Daten, insbesondere der Strahlungsdosis (MED), die der Kunde bei vergangenen Bräunungen bereits bekommen hat, und vom Hauttyp des Kunden.

Die Vorrichtung ist vorzugsweise in das Solarium (Sonnenbank) eingebaut. Bei einer besonders bevorzugten Ausführungsform mißt die Vorrichtung in gewissen zeitlichen Abständen die UV-Intensität. Der Kunde gibt über eine Tastatur seine Vorbräunung ein und erhält über die Datenausgabeeinheit hauttypabhängig drei mögliche Bräunungszeiten.

Der bei der erfindungsgemäßen Vorrichtung verwendete UV-Sensor ist vorzugsweise im Solarium in einem Gehäuse vor der zu vermessenden UV-Röhre befestigt. Vorzugsweise findet als UV-Sensor der Baustein UV 10-T2E-10F der Firma Perkinelmer Verwendung. Bei geringer Lichtleistung, bei der der integrierte Verstärker noch nicht übersteuert wird, darf der Sensor dauerhaft mit UV-Licht bestrahlt werden. Bei der Übertragung des UV-Lichtes durch eine sehr kleine Gehäuseöffnung erreicht ihn nur eine geringe Menge UV-Licht. Die UV-Lichtleistung kann auch durch einen Lichtwellenleiter begrenzt werden, über den die Übertragung der UV-Strahlung folgen kann. Es muß dabei jedoch darauf geachtet werden, daß der Lichtwellenleiter keinem Alterungsprozeß unterliegt, oder es müssen geeignete Maßnahmen ergriffen werden, die die Alterung verhindern bzw. reduzieren (beispielsweise Shutter-Mechanik). Der Lichtwellenleiter wird vorzugsweise direkt senkrecht vor der Röhre plaziert. Das andere Ende des Lichtwellenleiters wird unmittelbar vor dem UV-Sensor angebracht, so daß ein optimaler Empfang der strahlung gewährleistet ist.

Ein mechanischer Shutter ist nur für den Fall erforderlich, daß der UV-Sensor oder der Lichtwellenleiter einer starken Alterung durch dauerhafte UV-Licht-Bestrahlung unterliegt. Ein derartiger Shutter dichtet das Gehäuse der Lichtaufnahm-Optik nach außen hin ab. Die Shutter-Ansteuerung wird vorzugsweise durch den Microcontroller übernommen. Der Shutter öffnet sich nur während einer UV-Messung, ansonsten ist er dicht verschlossen und läßt keinen Lichteinfall zu.

Zweckmäßigerweise besitzt das verwendete Benutzerinterface eine Folientastatur mit vorzugsweise fünf Tasten, die die einzelnen Bedienelemente für Kunden und Personal darstellen. Hierüber stellt der Kunde seinen Bräunungsstatus ein. Dem Bedienungspersonal ermöglichen die Tasten des Zugang zum Statistikmenü (Betriebsstundenabfragen/Zurücksetzen u.a.).

Als Datenausgabeeinheit findet vorzugsweise ein LCD-Display Verwendung, das auch zur Anzeige einer laufenden Messung dienen kann. Die Ansteuerung erfolgt direkt durch den Microcontroller. Die entsprechenden Daten werden über einen Bus übermittelt.

Zusätzliche LEDs können zur Signalisierung einer durchgeführten Messung Verwendung finden.

Als Speicher findet vorzugsweise ein EEPROM Verwendung.

In die verwendete Software sind vorzugsweise folgende Funktionen eingebunden:
Durchführung einer Messung (Ansteuerüng des Shutters, zeitgesteuerte Messung)
Abspeicherung/Auslesen von Werten im Speicher (UV-Meßwerte, Betriebsstunden)
Bewegen im Benutzermenü (Abfrage des Hauttyps und der Bräunungsintensität)
Ansteuerung des Displays, Ausgabe der Werte, Abfragen von Fehlerständen
Abfrage der Eingänge (Bräunung gestartet, Tastatur) Bedienung des AD-Wandlers.

Wenn neben dem Sensor ein Lichtwellenleiter und ein Shutter Verwendung finden, so werden diese drei Komponenten vorzugsweise in einem gemeinsamen Gehäuse angeordnet. Dieses Gehäuse dient der Fixierung der drei Elemente, so daß die Messungen immer an einem fixen Punkt durchgeführt werden. Darüber hinaus schottet es die lichtempfindlichen Bauelemente zusätzlich von der starken UV-Strahlung ab. Der Shutter muß dafür sorgen, daß den Lichtwellenleiter und/oder den Sensor außerhalb einer Messung keine UV-Strahlung erreicht.

Die Erfindung wird nachfolgend anhand eines Ausführungsbeispiels in Verbindung mit der beigefügten Zeichnung im einzelnen erläutert. Es zeigen:
- Figur 1: ein Blockdiagramm, das schematisch den Aufbau einer Vorrichtung zum Ermitteln der optimalen Bräunungsdauer zeigt;
- Figur 2: eine schematische Ansicht einer Sonnenbank mit eingebauter Vorrichtung zum Ermitteln der optimalen Bräunungsdauer und teilweise entfernt dargestelltem Deckel; und
- Figur 3: eine schematische Darstellung des Benutzer-Interfaces.

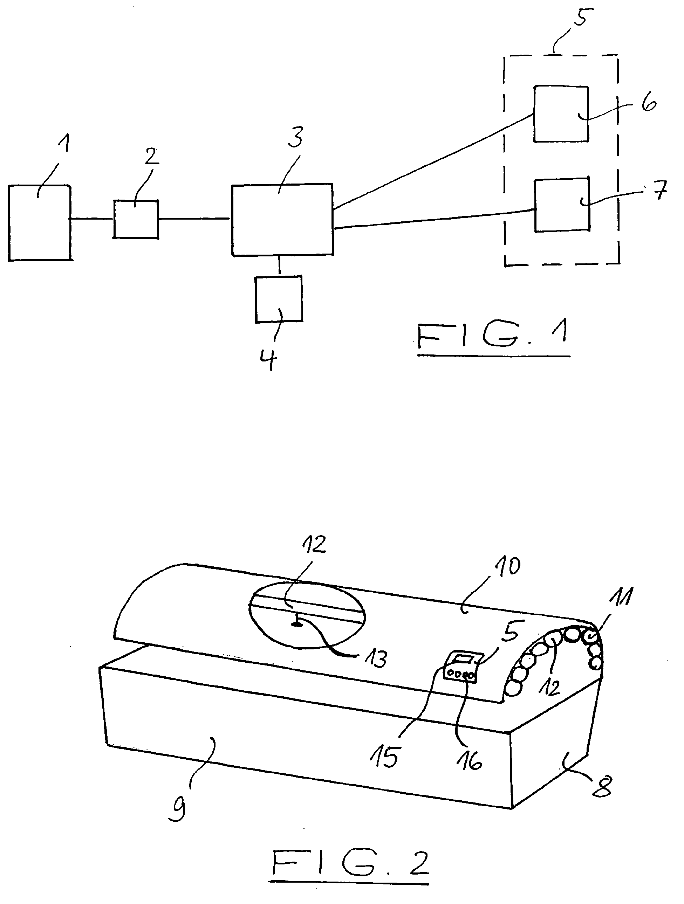

Die in dem Blockdiagramm der Figur 1 schematisch dargestellte Vorrichtung zum Ermitteln der Bräunungsdauer von Personen in Solarien besitzt einen UV-Sensor 1 zum Bestimmen der Strahlungsintensität des Solariums. Es handelt sich hierbei um einen Sensor mit Erythemcharakteristik, der beispielsweise von bekannter Bauart sein kann. Die vom Sensor in Abhängigkeit von der Strahlungsintensität abgegebenen analogen Spannungssignale werden einem A/D-Wandler 2 zugeführt und dort in digitale Signale umgewandelt. Die entsprechenden Signale werden einer in Form eines Mikrocontrollers ausgebildeten Zentraleinheit 3 zugeführt.

Der Sensor 1 führt periodisch, beispielsweise in Zeitabständen von 5 h, entsprechende Strahlungsintensitätsmessungen durch. Er wird diesbezüglich über die dargestellte Leitung von der Zentraleinheit 3 angesteuert.

Die vom UV-Sensor 1 auf die Zentraleinheit 3 übertragenen digitalen Meßsignale werden als Meßdaten in einem als EEPROM ausgebildeten Speicher 4 gespeichert. Die Zentraleinheit 3 liest die Daten in den Speicher ein und aus diesem aus.

Ferner ist die Zentraleinheit 3 über entsprechende bidirektionale Verbindungen an eine Dateneingabeeiheit 7 und eine Datenausgabeeinheit 6 angeschlossen, die Teil eines Benutzerinterfaces 5 bilden. Bei der Dateneingabeeinheit 7 handelt es sich um eine geeignete Tastatur, beispielsweise Folientastatur. Die Datenausgabe 106 ist als LCD-Display ausgebildet.

Über die Dateneingabeeinheit 7 kann der Benutzer der Vorrichtung personenspezifische Daten, beispielsweise Vorbräungsdaten, Hauttypdaten etc., in die Vorrichtung eingeben. Diese Daten werden der Zentraleinheit 3 zugeführt und dort zusammen mit den aus dem Speicher 4 ausgelesenen Meßdaten ausgewertet. Die Auswertung erfolgt mit Hilfe einer geeigneten Software. Ziel ist es, eine optimale Bräunungsdauer in Abhängigkeit von der UV-Strahlungsintensität des betreffenden Solariums sowie den eingegebenen personenspezifischen Daten zu ermitteln. Diese optimale Bräunungsdauer wird von der Zentraleinheit 3 errechnet und an der Datenausgabeeinheit 6 (LCD-Display) angezeigt.

Figur 2 zeigt eine Sonnenbank 8, in die die Vorrichtung der Figur 1 eingebaut ist. Der schematisch bei 13 dargestellte UV-Sensor ist der mittleren und damit am höchsten gelegenen UV-Röhre 12 des Deckelbereiches zugeordnet, und zwar mittig in Längsrichtung derselben. Der Sensor ist in einem geeigneten Gehäuse (nicht gezeigt) untergebracht und kann beispielsweise eine Shutter-Steuerung aufweisen, die die UV-Bestrahlung des Sensors durch Öffnen und Schließen des Gehäuses steuert. Dieser UV-Sensor 13 steht über eine Leitung mit der Steuereinheit der Vorrichtung in Verbindung, die die übrigen in Figur 1 gezeigten Teile umfaßt. Diese Steuereinheit ist in den Deckel eingebaut und in Figur 2 nicht zu erkennen, abgesehen von dem Benutzerinterface 5 mit Dateneingabeeinheit 7 und Datenausgabeeinheit 6. Man erkennt, daß das Benutzerinterface 5 infolge von dessen Anordnung an der Außenseite des Sonnenbankdeckels 10 im geschlossenen Zustand des Deckels 10, indem dieser auf dem Unterteil 9 aufliegt, bedient werden kann. Mit 11 sind die weiteren UV-Röhren des Deckels bezeichnet.

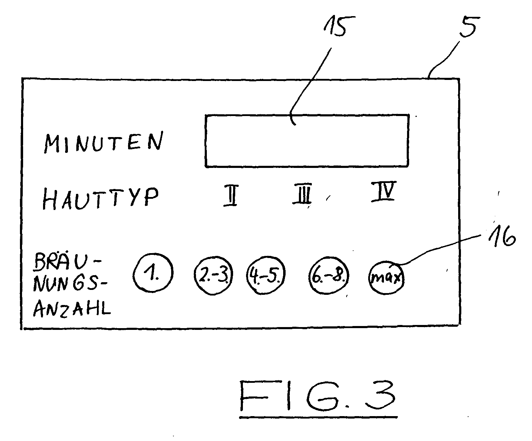

Figur 3 zeigt das Benutzerinterface 5 in vergrößerter Darstellung. Die Datenausgabeeinheit 6 ist hierbei als LCD-Display 15 schematisch angedeutet und zeigt drei Ziffern an, die jeweils einer optimalen Bräunungszeit für den Hauttyp II, III und IV entsprechen. Bei der Dateneingabeeinheit 7 handelt es sich um eine Folientastatur, die von fünf Tasten 16 gebildet wird. Durch Berühren dieser Tasten gibt die das Solarium nutzende Person die Zahl des jeweiligen Bräunungsvorganges ein, d.h. wenn die Person das Solarium zum erstenmal benutzt, drückt sie die mit 01. gekennzeichnete Taste, wenn sie es zum zweiten oder dritten Male benutzt, die mit 02. - 03. gekennzeichnete Taste etc.. Darüber hinaus kann eine Maximal-Taste berührt werden.

Entsprechende Schalter bzw. Tasten zum Ein-Ausschalten bzw. zur Inbetriebnahme der Vorrichtung sind nicht dargestellt.

## Patentansprüche

1. Vorrichtung zum Ermitteln der optimalen Bräunungsdauer von Personen in Solarien mit
a. einem UV-Sensor (1) zum Bestimmen der Strahlungsintensität des Solariums (8) und
b. einer Steuerungseinheit enthaltend
c. eine Dateneingabeeinheit (7) zum Eingeben von Eigenschaften (Vorbräunung, Hauttyp etc.) einer das Solarium (8) benutzenden Person kennzeichnenden Daten;
d. eine Datenausgabeeinheit (6) zum Ausgeben von die Bräunungsdauer kennzeichnenden Daten; und
e. eine Zentraleinheit (3) zum Ansteuern des UV-Sensors (1) und der Datenausgabeeinheit (6), zum Empfangen der Daten vom UV-Sensor (1) und der Dateneingabeeinheit (7) und zum Auswerten dieser Daten und Berechnen der Bräunungsdauerdaten.

2. Vorrichtung nach Anspruch 1, **dadurch gekennzeichnet, daß** sie einen Speicher (4) zum Speichern der gemessenen Strahlungintensitätsdaten aufweist.

3. Vorrichtung nach Anspruch 1 oder 2, **dadurch gekennzeichnet, daß** die Datenausgabeeinheit (6) eine optische Anzeige aufweist.

4. Vorrichtung nach einem der vorangehenden Ansprüche, **dadurch gekennzeichnet, daß** die Dateneingabeeinheit (7) eine Tastatur umfaßt.

5. Vorrichtung nach einem der vorangehenden Ansprüche, **dadurch gekennzeichnet, daß** die Zentraleinheit (3) als Microcontroller ausgebildet ist.

6. Vorrichtung nach einem der vorangehenden Ansprüche, **dadurch gekennzeichnet, daß** die Zentraleinheit (3) den UV-Sensor (1) derart ansteuert, daß dieser in zeitlichen Abständen mehrfach die Strahlungsintensität mißt.

7. Vorrichtung nach einem der vorangehenden Ansprüche, **dadurch gekennzeichnet, daß** es sich bei dem UV-Sensor (1) um einen UV-Intensitätssensor mit Erythemcharakteristik handelt.

8. Vorrichtung nach einem der vorangehenden Ansprüche, **dadurch gekennzeichnet, daß** das UV-Licht zum UV-Sensor (1) über einen Lichtwellenleiter übertragen wird.

9. Vorrichtung nach einem der vorangehenden Ansprüche, **dadurch gekennzeichnet, daß** der UV-Sensor einen Shutter aufweist.

10. Vorrichtung nach einem der vorangehenden Ansprüche, **dadurch gekennzeichnet, daß** Dateneingabeeinheit (7) und Datenausgabeeinheit(6) als Benutzerinterface (5) mit Folientastatur und LCD-Display ausgebildet sind.

11. Vorrichtung nach einem der vorangehenden Ansprüche, **dadurch gekennzeichnet, daß** sie an ein entferntes Terminal anschließbar ist.

12. Solarium, **dadurch gekennzeichnet, daß** es eine Vorrichtung nach einem der Ansprüche 1 - 11 enthält.

13. Solarium nach Anspruch 12, **dadurch gekennzeichnet, daß** die Steuerungseinheit in den Deckel (10) des Solariums (8) eingebaut oder an diesen angebaut ist.

14. Solarium nach Anspruch 12 oder 13, **dadurch gekennzeichnet, daß** der UV-Sensor (1,13) einer UV-Röhre (12) im Deckel (10) zugeordnet ist.
